(19) Europäisches Patentamt
European Patent Office
Office européen des brevets

(11) EP 0 850 976 A1

(12) EUROPÄISCHE PATENTANMELDUNG

(43) Veröffentlichungstag: **01.07.1998 Patentblatt 1998/27**

(51) Int. Cl.[6]: **C08G 73/02**

(21) Anmeldenummer: **97122116.3**

(22) Anmeldetag: **16.12.1997**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **23.12.1996 DE 19654058**

(71) Anmelder:
**BASF AKTIENGESELLSCHAFT**
**67056 Ludwigshafen (DE)**

(72) Erfinder:
- **Kratz, Detlef, Dr.**
  **69121 Heidelberg (DE)**
- **Lippert, Ferdinand, Dr.**
  **67098 Bad Dürkheim (DE)**
- **Schwab, Peter, Dr.**
  **67098 Bad Dürkheim (DE)**
- **Boeckh, Dieter, Dr.**
  **67117 Limburgerhof (DE)**
- **Perner, Johannes, Dr.**
  **67434 Neustadt (DE)**

# (54) Polymere Polyamine aus alternierenden Polyketonen

(57) Polymere Polyamine erhältlich durch Umsetzung von linearen, alternierenden Kohlenmonoxid/Alk-1-en-Copolymeren mit Ammoniak oder Aminen der allgemeinen Formel (I)

$$R—NH_2 \qquad (I)$$

worin R $-NH_2$, -OH, C1- bis $C_{10}$-Alkyl, $C_6$- bis $C_{20}$-Aryl, $C_7$- bis $C_{20}$-Arylalkyl, $C_7$- bis $C_{20}$ Alkylaryl oder ein siliciumorganischer Rest mit 3 bis 30 Kohlenstoffatomen bedeutet

oder einem Ammoniak oder die Amine (I) freisetzenden Reagenz und anschließende Hydrierung.

EP 0 850 976 A1

**Beschreibung**

Die vorliegende Erfindung betrifft polymere Polyamine erhältlich durch Umsetzung von linearen, alternierenden Kohlenmonoxid/Alk-1-en-Copolymeren mit Ammoniak oder Aminen der allgemeinen Formel (I)

$$R—NH_2 \quad (I)$$

worin R $-NH_2$,-OH, $C_1$- bis $C_{10}$-Alkyl, $C_6$- bis $C_{20}$-Aryl, $C_7$- bis $C_{20}$-Arylalkyl, $C_7$- bis $C_{20}$ Alkylaryl oder ein silicium-organischer Rest mit 3 bis 30 Kohlenstoffatomen bedeutet

oder einem Ammoniak oder die Amine (I) freisetzenden Reagenz und anschließende Hydrierung, ein Verfahren zur Herstellung von polymeren Polyaminen durch Umsetzung von Kohlenmonoxid/Alk-1-en-Copolymeren mit Ammoniak oder Aminen der allgemeinen Formel (I)

$$R—NH_2 \quad (I)$$

worin R $-NH_2$, -OH, $C_1$- bis $C_{10}$-Alkyl, $C_6$- bis $C_{20}$-Aryl, $C_7$- bis $C_{20}$-Arylalkyl, $C_7$- bis $C_{20}$ Alkylaryl oder ein silicium-organischer Rest mit 3 bis 30 Kohlenstoffatomen bedeutet

oder einem Ammoniak oder die Amine (I) freisetzenden Reagenz und anschließende Hydrierung, sowie die Verwendung der polymeren Polyamine als Tenside, Dispergiermittel, Papierhilfsmittel, Schmutzlösungsmittel, Komponente in Hautcremes und Haarpflege, Vernetzer für Klebstoffe, Stabilisator für Polyoxymethylen, Korrosionsinhibitoren, Textilhilfsmittel, Hilfsmittel für Dispersionen, Klebstoffe, Schutzkolloide, Haftbeschichtung, Epoxidharzhärter in wässrigen Dispersionen, Hilfsmittel für Geschirrspülmittel, Papierhilfsmittel, Egalisiermittel für Textilien, Solubilisiator für Kosmetika, Hilfsmittel für die Metallextraktion, Komplexbildner, Kraftstoffadditiv, Schmierstoffe, Korrosionsinhibitor für wässrige Systeme, Zusatz für Leim- und Harzrohstoffe, Hilfsmittel zur Farbstoffixierung auf Textilien, Hilfsmittel in der Papierfixierung, Retentionshilfsmittel, Komplexbildner für Metall-Recycling, Stabilisatoren für Hydroxylamin oder Farbübertragungsinhibitoren.

Polymere Polyamine sind wichtige Hilfsmittel beispielsweise in der Papier- oder Waschmittelindustrie. Sie werden im allgemeinen durch die Polymerisation von sticktoffhaltigen Monomeren erhalten. Die Eigenschaften der heute verfügbaren polymeren Polyamine werden in der Regel über die Struktur und Reaktivität der Monomeren bestimmt und müssen an unterschiedliche Verwendungszwecke angepaßt werden. Durch die geringe Anzahl von kommerziell verfügbaren Stickstoff-Monomeren ist diese Anpassung nur eingeschränkt möglich. Die Eigenschaften des Polymers werden üblicherweise neben dem Verhältnis N/C auch durch die Art der N-Funktion geprägt. So ist es wünschenswert, das molare Verhältnis von primären, sekundären oder tertiären N-Funktionen zu beeinflussen.

Aufgabe der vorliegenden Erfindung war es daher polymere Polyamine mit wechselnden Eigenschaften und einem relativ hohen Anteil an Amingruppen, möglichst mittels eines variablen Verfahrens, zur Verfügung zu stellen.

Daher wurden die polymere Polyamine, erhältlich durch Umsetzung von linearen, alternierenden Kohlenmonoxid/Alk-1-en-Copolymeren mit Ammoniak oder Aminen der allgemeinen Formel (I)

$$R—NH_2 \quad (I)$$

worin R $-NH_2$, -OH, $C_1$- bis $C_{10}$-Alkyl, $C_6$- bis $C_{20}$-Aryl, $C_7$- bis $C_{20}$-Arylalkyl, $C_7$- bis $C_{20}$ Alkylaryl oder ein silicium-organischer Rest mit 3 bis 30 Kohlenstoffatomen bedeutet

oder einem Ammoniak oder die Amine (I) freisetzenden Reagenz und anschließende Hydrierung, sowie ein Verfahren zur Herstellung von polymeren Polyaminen durch Umsetzung von Kohlenmonoxid/Alk-1-en-Copolymeren mit Ammoniak oder Aminen der allgemeinen Formel (I)

$$R—NH_2 \quad (I)$$

worin R $-NH_2$, -OH, $C_1$- bis $C_{10}$-Alkyl, $C_6$- bis $C_{20}$-Aryl, $C_7$- bis $C_{20}$-Arylalkyl, $C_7$- bis $C_{20}$ Alkylaryl oder ein silicium-organischer Rest mit 3 bis 30 Kohlenstoffatomen bedeutet

oder einem Ammoniak oder die Amine (I) freisetzenden Reagenz und anschließende Hydrierung wobei man lineare, alternierende Kohlenmonoxid/Alk-1-en-Copolymere verwendet gefunden. Weiterhin wurde die Verwendung der eingangs definierten polymeren Polyamine unter anderem als Tenside und Farbübertragungsinhibitoren gefunden.

Die den polymeren Polyaminen zugrunde liegenden linearen, alternierenden Kohlenmonoxid/Alk-1-en- Copolymere (alternierende Polyketone) sind bekannt und im allgemeinen durch palladiumkatalysierte Copolymerisation von Kohlenmonoxid mit einem Alk-1-en oder mehreren Alk-1-enen erhältlich. In der Regel weisen sie folgende Wiederholeinheit auf

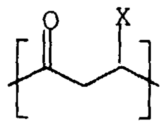

II.

Hierin bedeutet X Wasserstoff, $C_1$- bis $C_{10}$-Alkyl, wie Methyl, Ethyl n-Propyl, iso-Propyl, n-Butyl, tert.-Butyl, n-Hexyl, n-Octyl oder n-Decyl, vorzugsweise jedoch Wasserstoff oder Methyl. Die linearen alternierenden Copolymeren umfassen somit auch Polymere mit mehr als einer Sorte vom Alk-1-en abgeleitete Struktureinheiten wie Kohlenmonoxid/Ethylen/Propen-Terpolymere. Das molare Verhältnis Carbonylgruppe : Alkylenstruktureinheit der alternierenden Polyketone beträgt üblicherweise 1 : 1. Das mittlere Molekulargewicht Mn der linearen, alternierenden Kohlenmonoxid/Alk-1-en-Copolymeren, bestimmt mit der Methode der NMR-Spektroskopie, liegt üblicherweise im Bereich von 100 bis 1 000 000, vorzugsweise im Bereich von 150 bis 500 000, insbesondere im Bereich von 100 bis 50 000.

Die erfindungsgemäßen polymeren Polyamine können durch die Umsetzung der beschriebenen linearen, alternierenden Kohlenmonoxid/Alk-1-en-Copolymeren mit Ammoniak, Aminen der allgemeinen Formel $R\text{-}NH_2$ (I) oder einem Ammoniak oder die Amine (I) freisetzenden Reagenz und anschließende Hydrierung erhalten werden.

Als Reste R in (I) kommen $-NH_2$, -OH, $C_1$- bis $C_{10}$-Alkyl, wie Methyl, Ethyl, n-Propyl, i-Propyl, n-Butyl, sec.-Butyl, iso-Butyl, tert.-Butyl, n-Hexyl, n-Octyl, n-Decyl weiterhin $C_6$- bis $C_{20}$-Aryl, wie Phenyl, Naphthyl, $C_7$- bis $C_{20}$-Arylalkyl, wie Benzyl und $C_7$- bis $C_{20}$ Alkylaryl, wie p-Tolyl, o-Tolyl, Xylyl in Betracht. Geeignete Reste R in (I) sind weiterhin siliciumorganische Reste mit 3 bis 30 Kohlenstoffatomen, mithin Tri($C_1$- bis $C_{10}$)alkylsilyl, wie Trimethylsilyl, Tert.-Butyldimethylsilyl oder auch Triarylsilyl, beispielsweise Triphenylsilyl, Tri-p-tolylsily oder Trinaphthylsilyl.

Als Ammoniak freisetzende Reagenzien kommen generell alle Ammoniumsalze in Frage, vorzugsweise Ammoniumcarbonat, Ammoniumacetat, Ammoniumformiat.

Vorzugsweise verwendet man Ammoniak, Hydrazin, Ammoniumcarbonat, gegebenfalls in Form der Hydrate, als Aminierungsreagenz. Ammoniak ist ganz besonders bevorzugt. Ammoniak, die Amine (I) oder die Ammoniak oder die Amine (I) freisetzenden Reagenzien, im folgenden Aminierungsreagenzien genannt, können als Reinsubstanzen oder gelöst in organischen Lösungsmitteln, wie Tetrahydrofuran, Dioxan, Toluol, N-Methylpyrrolidon oder Wasser oder deren Mischungen eingesetzt werden.

Die Aminierungsreagenzien können sowohl im Überschuß, stöchiometrisch oder aber unterstöchiometrisch, jeweils bezogen auf die Carbonylgruppe >C=O der linearen, alternierenden Kohlenmonoxid/Alk-1-en-Copolymeren eingesetzt werden.

Die bevorzugte Umsetzung der linearen alternierenden Kohlenmonoxid/Alk-1-en-Copolymeren mit stöchiometrischen Mengen oder einem Überschuß Ammoniaks führt üblicherweise nach Hydrierung zu polymeren Polyaminen mit einem molaren Verhältnis von primärer Aminfunktion : (Summe aus sekundärer Aminfunktion und tertiärer Aminfunktion) im Bereich von 1 : 1 bis 1 : 10 000, vorzugsweise 1 : 2 bis 1 : 100. Die Bestimmung der diesen Verhältnissen zugrunde liegenden Aminzahlen geschieht durch Titration, wie in den Beispielen beschrieben.

Nach gegenwärtigem Kenntnisstand geht man davon aus, daß in den so erhältlichen polymeren Polyaminen die folgenden Struktureinheiten III vorliegen können, wobei die, durch die Indizes a,b und c gekennzeichneten Struktureinheiten in der Regel statistisch über die Polymerkette verteilt sind.

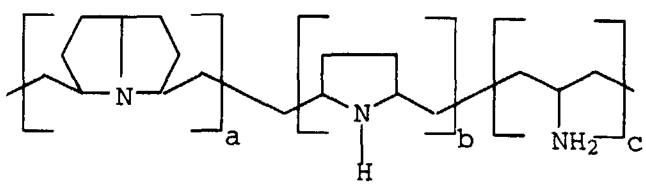

III

Besonders gute Ergebnisse werden in der Regel dann erzielt, wenn das molare Verhältnis Aminierungsreagenz : Carbonylgruppe des Polyketons im Bereich von 1,1 : 1 bis 20 : 1 liegt oder insbesondere 2 : 1 beträgt.

Die mit stöchiometrischen Mengen oder mit einem Überschuß an Aminierungsreagenzien erhältlichen polymeren

Polyamine enthalten im allgemeinen weniger als 5 Gew.-% Carbonylgruppen >C=O bezogen auf das polymere Polyamin, bestimmt mit der Methode der $^{13}C$-NMR-Spektroskopie bei 20°C in $CDCl_3$, und weniger als 10 Gew.-%, vorzugsweise weniger als 1 Gew.-%, bezogen auf das polymere Polyamin, Hydroxylgruppen.

Werden die Aminierungsreagenzien in unterstöchiometrischen Mengen eingesetzt, vorzugsweise in Mengen im Bereich von 0,01 bis 0,99, bezogen auf die Carbonylfunktion >C=O im Polyketon so erhält man im allgemeinen polymere Polyamine, die eine Etherfunktion, als 2,5-Tetrahydrofurandiyleinheit, nachgewiesen mit der Methode der $^{13}C$NMR Spektroskopie, sowie Hydroxylgruppen enthalten. Die Mengen und molaren Verhältnisse der funktionellen Stickstoff- und Sauerstoffgruppen im polymeren Polyamin hängen üblicherweise von den Reaktionsbedingungen bei deren Herstellung mit unterstöchiometrischen Mengen Aminierungsreagenzien ab. Üblicherweise liegt das molare Verhältnis Gesamtmenge chemisch gebundener Stickstoff im polymeren Polyamin : Gesamtmenge chemisch gebundener Ether-und Hydroxylsauerstoff im polymeren Polyamin im Bereich von 1 : 1000 bis 1000 : 1, vorzugsweise im Bereich von 1 : 100 bis 100 : 1.

Bei der besonders bevorzugten Umsetzung von unterstöchiometrischen Mengen Ammoniaks, vorzugsweise in Mengen im Bereich von 0,1 bis 0,99, bezogen auf die Carbonylfunktion >C=O, mit den linearen alternierenden Kohlenmonoxid/Alk-1-en-Copolymeren, vorzugsweise Kohlenmonoxid/Ethylencopolymeren, erhält man im allgemeinen polymere Polyamine, die zusätzlich zu den bereits beschriebenen primären, sekundären und tertiären Aminfunktionen eine Etherfunktion, als 2,5-Tetrahydrofurandiyleinheit, sowie Hydroxylgruppen enthalten. Die Mengen und molaren Verhältnisse der funktionellen Stickstoff- und Sauerstoffgruppen im polymeren Polyamin hängen auch hier üblicherweise von den Reaktionsbedingungen bei der Herstellung der polymeren Polyamine mit unterstöchiometrischen Mengen Ammoniaks ab. Üblicherweise liegt das molare Verhältnis Gesamtmenge chemisch gebundener Stickstoff im polymeren Polyamin : Gesamtmenge chemisch gebundener Ether- und Hydroxylsauerstoff im polymeren Polyamin im Bereich von 1 : 1000 bis 1000 : 1, vorzugsweise im Bereich von 1 : 100 bis 100 : 1. Das molare Verhältnis primäre Aminfunktion : (sekundäre Aminfunktion + tertiäre Aminfunktion) im polymeren Polyamin liegt üblicherweise im Bereich von 1 : 1 bis 1 : 10000, vorzugsweise im Bereich von 1 : 2 bis 1 : 100.

Die mit unterstöchiometrischen Mengen Aminierungsreagenzien erhältlichen polymeren Polyamine enthalten im allgemeinen weniger als 5 Gew.-%, vorzugsweise weniger als 3 Gew.-% Carbonylgruppen >C=O, bezogen auf das polymere Polyamin, bestimmt mit der Methode der 13C-NMR-Spektroskopie bei 20°C in $CDCl_3$ als Lösungsmittel.

Das mittlere zahlenmäßige Molekulargewicht Mn, bestimmt mit der Methode der Gelpermeationschromatographie, der erfindungsgemäßen polymeren Polyamine liegt im allgemeinen im Bereich von 100 bis 1 000 000, vorzugsweise im Bereich von 100 bis 500 000 und insbesondere im Bereich von 100 bis 50 000.

Ein geeignetes Verfahren zur Herstellung der polymeren Polyamine ist die Umsetzung der eingangs definierten linearen alternierenden Kohlenmonoxid/Alk-1-en-Copolymeren mit Ammoniak oder den bereits definierten Aminen R-$NH_2$ (I) oder einem bereits definierten Ammoniak oder die Amine (I) freisetzenden Reagenz und anschließende Hydrierung.

Als besonders geeignet erwiesen hat sich ein zweistufiges Verfahren in welchem man in der ersten Stufe die linearen alternierenden Kohlenmonoxid/Alk-1-en-Copolymeren mit Ammoniak oder den bereits definierten Aminen R-$NH_2$ (I) oder einem Ammoniak oder die Amine (I) freisetzenden Reagenz, üblicherweise bei einer Temperatur im Bereich von 20 bis 250°C, vorzugsweise im Bereich von 50 bis 150°C und einem Druck im Bereich von 100 bis 20 000 kPa in Sübstanz oder gegebenenfalls in Wasser oder organischen Lösungsmittel, wie Tetrahydrofuran, Dioxan, Toluol, N-Methylpyrrolidon, Cyclohexan umsetzt. In einer zweiten Stufe wird dann im allgemeinen - vorzugsweise ohne Isolierung und Aufarbeitung der Reaktionsprodukte der ersten Stufe - in Gegenwart eines Hydrierkatalysators bei einem Wasserstoffdruck im Bereich von 100 bis 35 000 kPa, vorzugsweise 1000 bis 25 000 kPa und einer Temperatur im Bereich von 20 bis 250°C, vorzugsweise im Bereich von 100 bis 220°C die Hydrierung durchgeführt.

Als Hydrierkatalysator kommen alle hierfür in D.D. Coffmann, J. Am. Chem. Soc. 76 (1954), 6394-6399; A. Sen, Adv. Polym. Sci. (1986) 125-144 beschriebenen in Frage, vorzugsweise verwendet man jedoch Nickel (14 Gew.-%)/Aluminiumoxid, Ruthenium (0,5 Gew.-%)/makroporösem Aluminiumoxid oder Rutheniumoxidhydrat.

Üblicherweise werden die Aminierung und die Hydrierung solange durchgeführt, bis mindestens 5 mol-% der Carbonylgruppen >C=O des Ausgangspolyketons umgesetzt sind, welches mittels Infrarotspektroskopie überprüft werden kann. Bevorzugte Reaktionszeiten, sowohl für die Aminierung als auch für die Hydrierung, liegen im Bereich von 0,5 bis 20 Stunden.

Die Aminierungen können, wie bereits beschrieben, mit einem Überschuß, stöchiometrischen Mengen oder mit unterstöchiometrischen Mengen Aminierungsreagenz, vorzugsweise Ammoniaks, bezogen auf die Carbonylgruppe >C=O des linearen alternierenden Kohlenmonoxid/Alk-1-en-Copolymeren durchgeführt werden.

Die erfindungsgemäßen polymeren Polyamine lassen sich noch mit den üblichen Methoden der Chemie modifizieren. Beispielsweise führt die Umsetzung mit Säuren, wie Carbonsäuren, also Essigsäure, Trifluoressigsäure oder auch anorganischen Säuren, wie Schwefelsäure, Salzsäure, Phosphorsäure zu den Ammoniumsalzen.

Ferner können die erfindungsgemäßen polymeren Polyamine mit $C_1$- bis $C_{25}$-Alkylierungsmitteln, wie Dialkylsulfaten, vorzugsweise Dimethylsulfat oder Diethylsulfat oder auch Alkylhalogeniden wie Benzylchlorid alkyliert werden.

Man verwendet hierfür im allgemeinen 0,1 bis 2 Moläquivalente, vorzugsweise 1 bis 2 Moläquivalente, Alkylierungsmittel, bezogen auf die im Polymeren vorhandene Amingruppen. Die Umsetzung der erfindungsgemäßen polymeren Polyamine mit Alkylenoxiden, vorzugsweise Ethylenoxid oder Propylenoxid führt üblicherweise zu den entsprechenden Alkoxylaten, z.B. Ethoxylaten.

Die erfindungsgemäßen polymeren Polyamine eignen sich gut als Tenside und vor allem als Farbübertragungsinhibitoren, besonders in Waschmitteln.

Die erfindungsgemäßen polymeren Polyamine sind insbesondere geeignet für aniontensidfreie Waschmittel und Wäschenachbehandlungsmittel und solche, die einen Aniontensidgehalt von weniger als 2,5 %, vorzugsweise weniger als 1,5 % besonders bevorzugt 0 bis 1 % Aniontenside aufweise.

Die erfindungsgemäßen polymeren Polyamine erreichen üblicherweise in aniontensidfreier Formulierung bereits bei einer Konzentration von 20 bis 100 ppm in der Wasch- bzw. Spülflotte das Wirkungsplateau, während Polymere nach dem Stand der Technik in Konzentrationen von ca. 500 ppm eingesetzt werden.

Die erfindungsgemäßen polymeren Polyamine werden im allgemeinen in den Wäschenachbehandlungsmitteln oder Waschmitteln zu 0,05 bis 10 %, vorzugsweise 0,1 bis 5,0 %, besonders bevorzugt 0,2 bis 2,5 %, bezogen auf die übrigen Formulierungsbestandteile eingesetzt.

Die erfindungsgemäßen polymeren Polyamine können vorteilhaft als Zusatz zu flüssigen oder festen Aniontensidfreien oder -armen Waschmittelformulierungen oder Wäschenachbehandlungsmitteln eingesetzt werden. Die Zusammensetzung von Waschmitteln und Wäschenachbehandlungsmitteln kann dabei in weiten Grenzen variiert werden.

## Beispiele

A) 1-H-NMR-spektroskopische Bestimmung des mittleren Molekulargewichts $M_n$ von Polyketon

Zur Bestimmung der Molmasse $M_n$ wurde von einer konzentrierten Probe Polyketon, gelöst in Hexafluorisopropanol : d6-Benzol (90 : 10), unter quantitativen Bedingungen, ein 1H-NMR-Spektrum aufgenommen (D1= 20 sek.; NS= 3000).

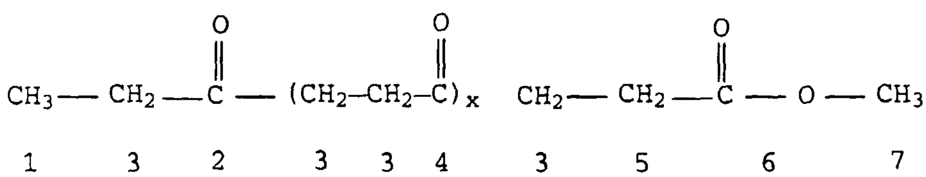

Aus den Integrationsstufenhöhen der Signale 1, 3 und 5 (evtl. auch 7) ließ sich die Molmasse berechnen.

Zuerst wurde der Mittelwert aus den Integrationsstufenhöhen der Signale 1 und 5 gebildet (ein Kohlenstoffatom entspricht x mm). Anschließend wurden die Integrationsstufenhöhen von Signal 3 $(-CH_2-CH_2-)_x$ durch den Mittelwert von C- der Endgruppe dividiert. Die erhaltene Anzahl von $CH_2$-Gruppen wurde durch 2 dividiert (minus Eine-Einheit=$2CH_2$ von Endgruppen) und man erhielt die Einheiten $CH_2$-$CH_2$-C=O.

Die Anzahl Einheiten wurden mit dem Molgewicht (~56) für eine Einheit multipliziert. Anschließend die Molmasse für die beiden Endgruppen

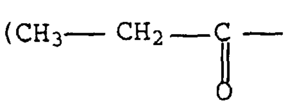 und 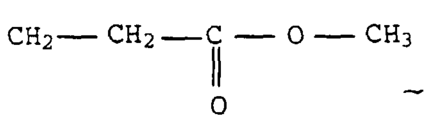 ~144 MG)

zuaddiert.

Der erhaltene Wert entsprach der mittleren Molmasse.

B) Bestimmung des Gesamtbasengehalts an Stickstoff

Ca. 150 mg der Probe wurden genau eingewogen und in 50 ml Eisessig gelöst. Diese Lösung wurde mit einer 0,1 mol/l Trifluormethansulfonsäuremaßlösung auf den Wendepunkt potentiographisch titriert.

Der prozentuale Gesamtbasengehalt an Stickstoff wurde nach folgender Formel berechnet:

$$w = c \cdot t \cdot M \cdot 0{,}1 \cdot (v1 - v2) / (m \cdot z)$$

wobei

c= molare Konzentration der Trifluormethansulfonsäurelösung,
t= Titer der Trifluormethansulfonsäurelösung
M= Molare Masse Stickstoff (14,01 g/mol)
v1= Volumenverbrauch in ml Trifluormethansulfonsäure für die Probe
v2= Volumenverbrauch in ml Trifluormethansulfonsäure für die Blindprobe
m= Probeneinwaage in g
z= Äquivalentzahl

bedeutet.

C) Bestimmung von tert. Amin

Ca. 150 mg Probe wurden genau gewogen, in 20 ml Essigsäure und 30 ml Essigsäureanhydrid gelöst und 2 h auf 70°C erwärmt. Nach Abkühlen wird potentiographisch auf den ersten Wendepunkt mit einer 0,1 mol/l Trifluormethansulfonsäurelösung titriert.

Die Berechnung des prozentualen Gehalts an tert. Amin erfolgt wie unter B) beschrieben.

D) Bestimmung von prim. Amin

Ca. 1,5 g der Probe wurden in 5 ml Pyridin gelöst, mit 5 ml Acetylaceton versetzt und 2 Stunden bei Raumtemperatur gerührt. Danach gab man noch 40 ml Pyridin und 10 ml Methanol zu und titrierte potentiographisch mit einer 1 mol/l Natriummethylatlösung zum Wendepunkt.

Die Berechnung des prozentualen Gehalts an prim. Amin erfolgte wie unter B) beschrieben.

E) Berechnung von sek. Amin

Man substrahierte vom Gesamtbasengehalt an Stickstoff der Probe die Summe der Werte für tert. Amin und prim. Amin, wie nach C) und D) bestimmt.

Beispiel 1

Herstellung eines alternierenden Polyketons

In einem 0,3 l-Autoklaven wurden Methanol, Palladiumacetat, Bis(di-tert.-butylphosphino)methan und p-Toluolsulfonsäure vorgelegt. Danach wurde bei der gewählten Reaktionstemperatur ein Gemisch aus Ethylen und Kohlenmonoxid im Molverhältnis 1:1 zu einem Gesamtdruck von 60 bar aufgepreßt. Temperatur und Partialdrücke der Monomeren wurden während der gesamten Reaktionsdauer konstant gehalten, wonach die Polymerisation durch Entspannen des Autoklaven abgebrochen und das Lösungsmittel abfiltriert wurde.

Die Verfahrensparameter, eingesetzte Mengen an Katalysator und Lösungsmittel sowie die erhaltenen Mengen an Copolymerisat und sein Schmelzpunkt sind der Tabelle zu entnehmen.

| | Beispiel |
|---|---|
| | 1 |
| Methanol [g] | 100 |
| Palladiumacetat [mg] | 49 |
| Bisphosphin [mg] | 270 |
| p-Toluolsulfonsäure [mg] | 140 |
| Reaktionstemperatur [°C] | 85 |
| Reaktionszeit [h] | 3 |
| Polyketon [g] | 43 |

(fortgesetzt)

| | Beispiel |
|---|---|
| | 1 |
| Schmelzpunkt [°C] | 237 |

Beispiel 2

146 g eines Polyketons der Molmasse Mn = 2600 wurden in 4000 g THF in einem Autoklaven mit 10 l Volumen suspendiert. Es wurden 8 g $RuO_2(H_2O)_6$ (Wassergehalt 75 %) hinzugegeben. Der Autoklav wurde verschlossen und unter Rühren wurde 600 g $NH_3$ aufgepreßt. Anschließend wurde der Autoklav unter Rühren auf 150°C erwärmt, wobei sich ein Eigendruck von 19 bar einstellte. Nach 15 h wurde Wasserstoff bei gleicher Temperatur bis 150 bar aufgepreßt. Anschließend wurde der Autoklav auf 200°C erwärmt und Wasserstoff auf 250 bar nachgepreßt. Die Reaktionszeit betrug 20 h. Der Autoklav wurde abgekühlt und entspannt, wobei überschüssiger Ammoniak entwich. Die hellgelbe Lösung wurde filtriert und das THF und gebildetes Wasser im Vakuum abdestilliert. Nach dem Trocknen an der Ölpumpe verblieben 93 g eines honigartigen Oligomers.

Kennzahlen:

| C | H | N | O |
|---|---|---|---|
| 75,6 | 11,2 | 11,2 | 1,9 |

primär N [g/100 g] 1,0]
sekundär N [g/100 g] 4,1]
tertiär N [g/100 g] 5,58]
gesamt N [g/100 g] 10,69]
OH-Zahl [mg KOH/g] 35]
CO-Zahl [mg KOH/g] 59]

Beispiel 3 bis 8

Die Reaktionen wurden wie in Beispiel 2 durchgeführt, jedoch wurden Polyketone unterschiedlicher Kettenlänge und unterschiedliche Mengenverhältnisse eingesetzt. Bedingungen und Ergebnisse sind in Tabelle 1 zusammengefaßt.

Beispiel 9 bis 13

Die Reaktion wurde wie in Beispiel 2 durchgeführt, jedoch wurde anstatt $Ru{=}_2(H_2O)_6$ als Katalysator ein heterogener Katalysator Ru (0,5 %) auf $Al_2O_3$ verwendet. Dieser war in einem Korb in dem Autoklaven eingetaucht. Bedingungen und Ergebnisse sind in Tabelle 2 zusammengefaßt.

Beispiel 14 bis 16

Die Reaktion wurde wie in Beispiel 9 durchgeführt, jedoch wurde Ammoniak unterstöchiometrisch eingesetzt (Tabelle 2).

Beispiel 17

Die Reaktion wurde wie in Beispiel 14 durchgeführt, jedoch wurden auf 168 g eingesetztem Polyketon zur Aminierung anstatt Ammoniak 24 g $(NH_4)_2CO_3$ verwendet (Tabelle 2).

Beispiel 18

100 g aminierend hydriertes Polyketon aus Beispiel 10 wurden bei 150 bis 155°C mit 5,5-Äquivalenten Ethylenoxid (EO) umgesetzt. Die im Austrag gefundene Menge (218 g) entspricht zu 97 % den eingesetzten Edukten (100 g aminierend hydriertes Polyketon und 124 g EO). Zur Entfernung von gebildeten Polyethylenglykolen wurde zweimal mit Diethylether extrahiert. Es verblieben 197 g Oligomer. Die Kennzahlen (nur noch tert. N-Funktionen) belegen, daß die Addition an alle N-Funktionen vollständig verlaufen ist.

| | aminierend hydriertes Polyketon (Beispiel 10) | Ethoxylat |
|---|---|---|
| OH-Zahl (mg KOH/g) | 178 | 283 |
| CO-Zahl (mg KOH/g) | 3 | 3 |
| prim. N (g/100 g) | 0,3 | <0,1 |
| sek. N (g/100 g) | 6,3 | <0,1 |
| tert. N (g/100 g) | 4,51 | 5,59 |
| ges. N (g/100 g) | 11,15 | 5,5 |

Beispiel 19

20 g aminierend hydriertes Polyketon aus Beispiel 5 wurden in 169,5 g wässriger Schwefelsäure (0,5 M) gelöst. Der pH-Wert der Lösung beträgt 3. Die Lösung wurde filtriert und das Wasser wurde im Vakuum abdestilliert. Es verblieben 29 g eines gut wasserlöslichen Feststoffs, der dem Sulfatsalz des Polymers entspricht.

Beispiel 20

Zu einer Lösung von 50 g aminierend hydriertem Polyketon aus Beispiel 5 in 450 g Toluol wurde bei 50 bis 70°C 82 g Dimethylsulfat gelöst in 82 g Toluol innerhalb von 1 h zugetropft. Es entstand ein brauner Feststoff, der abfiltriert wurde. Zur Entfernung von Überschuß Dimethylsulfat wurde der Feststoff in Wasser gelöst und mit Diethylether extrahiert. Die wässrige Phase wurde eingedampft und es verblieben 87 g Feststoff. Die Kennzahlen (s.u.) belegen, daß die Reaktion quantitativ alle primären, sekundären und tertiären N-Atome in quaternäre N-Atome überführt hatte.

| | aminierend hydriertes Polyketon (Beispiel 2) | Quaternisiertes Produkt |
|---|---|---|
| OH-Zahl (mg KOH/g) | n.b. | 140 |
| CO-Zahl (mg KOH/g) | n.b. | 94 |
| prim. N (g/100 g) | 0,2 | <0,1 |
| sek. N (g/100 g) | 6,47 | <0,1 |
| tert. N (g/100 g) | 4,77 | <0,01 |
| ges. Basen-N | 11,4 | <0,01 |
| quat. N | - | 1,1 |

Anwendungstechnische Beispiele

Die erfindungsgemäßen polymeren Polyamine oder deren Derivate aus den Beispielen 5, 6, 20 oder 19 wurden zur Prüfung der farbübertragungsinhibierenden Wirkung einem handelsüblichen Wäscheweichspüler zugesetzt und Farbgewebe vorgespült. Die Gewebe wurden mit` Leitungswasser nachgespült, getrocknet und gebügelt.

Anschließend wurden die vorbehandelten Farbgewebe mit weißen Prüfgeweben zusammen mit einem kommerziellen Waschmittel gewaschen. Die Farbstärke der weißen Prüfgewebe im Vergleich zur vorher gemessenen Farbstärke wurde gemessen, nach dem in A. Kud, Seifen, Öle, Fette, Wachse 119, S. 590-594 beschriebenen Verfahren die jeweiligen Farbstärken der Anfärbungen bestimmt und in dem beschriebenen Verfahren daraus die farbübertragungsinhibierende Wirkung ermittelt. Die Ergebnisse sind in Tabelle 3 aufgeführt.

Der Farbverlust der gefärbten Gewebe wurde durch Bestimmung der Farbstärke der Gewebe vor der ersten Wäsche und nach der 5. Wäsche bestimmt. Die %-Angaben sind
(Farbstärke vor der Wäsche - Farbstärke nach der fünften Wäsche)/Farbstärke vor der Wäsche

Prüfbedingungen:

Apparatur: Launder-o-meter
Farbgewebe: 1,0 g gefärbte Baumwollgewebe, Färbungen mit Direkt Rot 212 (3 % Farbstoff auf dem Gewebe)
Weißgewebe: 2,5 g Baumwollgewebe

Vorbehandlung:

Weichspüler: Softlan® (Hersteller Colgate-Palmolive) Einsatzkonzentration der Polymeren im Weichspüler: 2,0 %
Einsatzmenge Weichspüler: 1,75 g/l
Temperatur (Spülung): 30°C
Spüldauer: 10 min.

Wäsche:

Waschmittel: Ajax® (Hersteller Colgate-Palmolive)
Menge: 5,0 g/l
Flottenmenge: 250 g
Waschtemperatur: 40°C
Wasserhärte: 14,5°dH
Ca/Mg-Verhältnis: 4,0 : 1,0
Waschdauer: 30 min.

Tabelle 3

| Prüfergebnisse zur Farbübertragungsinhibierung und zur Minderung des Farbverlustes | | | |
|---|---|---|---|
| Versuchsserie | Polymer aus Beispiel | Farbübertragungsinhibierung in % bei der 1. Wäsche | Farbablösung in % nach 5 Wäschen |
| A | ohne | 0,0 | 30,5 |
| A | 5 | 98,0 | 7,8 |
| | | | |
| B | ohne | 0,0 | 13,3 |
| B | 6 | 84,0 | 8,7 |
| B | 20 | 93,2 | 8,7 |
| B | 19 | 93,0 | 4,0 |

Die Ergebnisse mit den erfindungsgemäß zu verwendenden Polymeren zeigen, daß die Polymeren bei Zusatz zu einem kommerziellen Weichspüler eine sehr gute farbübertragungsinhibierende und farbablösungsvermindernde Wirkung zeigen.

Tabelle 1a: Versuchsparameter

| Beisp. | Polyketon | | Lösungsmittel | | $NH_3$ | Katalysator | |
|---|---|---|---|---|---|---|---|
| | Molmasse (g/mol) | Masse (g) | | Menge (g) | Menge (g) | | Menge (g) |
| 2 | 2600 | 146 | THF | 4000 | 600 | $RuO_2$ 1) | 8 |
| 3 | 2000 | 250 | THF | 4000 | 600 | $RuO_2$ | 8 |
| 4 | 2000 | 500 | THF | 4000 | 1200 | $RuO_2$ | 12 |
| 5 | 2000 | 1000 | THF | 4000 | 1500 | $RuO_2$ | 24 |
| 6 | 2000 | 1000 | THF | 4000 | 1200 | $RuO_2$ | 24 |
| 7 | 11000 | 50 | THF | 1000 | 30 | $RuO_2$ | 5 |
| 8 | 10000 | 93 | THF | 1500 | 60 | $RuO_2$ | 7 |

1) $RuO_2$: $RuO_2(H_2O)_6$

noch Tabelle 1a: Versuchsparameter

| Beisp. | Reaktionsbedingungen | | | | | Auswaage | | Ausbeute[2] |
|---|---|---|---|---|---|---|---|---|
| | Iminierung | | Hydrierung | | | löslich | Auswaage/ | |
| | t (h) | T (°C) | t (h) | T (°C) | p (bar) | (g) | Einwaage | |
| 2 | 15 | 150 | 24 | 200 | 250 | 93 | 0,64 | 71,3 |
| 3 | 5 | 150 | 15 | 200 | 250 | 174 | 0,70 | 78,0 |
| 4 | 7 | 150 | 20 | 200 | 250 | 402 | 0,80 | 89,9 |
| 5 | 7 | 150 | 20 | 200 | 250 | 643 | 0,64 | 72,0 |
| 6 | 7 | 150 | 20 | 200 | 250 | 820 | 0,82 | 91,8 |
| 7 | 15 | 150 | 15 | 200 | 250 | 39 | 0,78 | 87,4 |
| 8 | 15 | 150 | 5 | 200 | 250 | 78 | 0,84 | 93,9 |

2) Ausbeute definiert durch Mol Produkt (= Auswaage/46) dividiert durch Mol Edukt (= Einwaage/56)

Tabelle 1b

| Stoffeigenschaften | | | | | | | | | | |
|---|---|---|---|---|---|---|---|---|---|---|
| Beispiel | Kennzahlen | | | | | | Elementaranalyse | | | |
| | OH | CO | prim. N | sek. N | tert. N | ges. N | C | H | N | O |
| | (mg KOH/g) | | (g/100 g) | | | | (g/100 g) | | | |
| 2 | 35 | 59 | 1,0 | 4,10 | 5,58 | 10,69 | 75,6 | 11,2 | 11,2 | 1,9 |
| 3 | 34 | 18 | 1,2 | 3,30 | 6,20 | 10,72 | 76,4 | 11,5 | 10,9 | 1,8 |
| 4 | n.b. | n.b. | 0,3 | 3,78 | 0,97 | 5,04 | n.b. | | | |
| 5 | 183 | <1 | 0,3 | 5,69 | 4,91 | 10,89 | n.b. | | | |
| 6 | n.b. | n.b. | 0,2 | 6,47 | 4,77 | 11,40 | n.b. | | | |
| 7 | 11 | <1 | 2,7 | 3,60 | 4,17 | 10,47 | n.b. | | | |
| 8 | <1 | <1 | 1,4 | 6,90 | 3,44 | 11,70 | n.b. | | | |
| n.b.: nicht bestimmt | | | | | | | | | | |

Tabelle 2a: Versuchsparameter

| Beisp. | Polyketon | | Lösungsmittel | | $NH_2$ | Katalysator | |
|---|---|---|---|---|---|---|---|
| | Molmasse (g/mol) | Masse (g) | | Menge (g) | Menge (g) | | Menge (g) |
| 9 | 2000 | 250 | THF | 1600 | 500 | Ru-Kat[1)] | 400 |
| 10 | 2000 | 250 | THF | 1000 | 300 | Ru-Kat | 400 |
| 11 | 2000 | 250 | THF | 1000 | 300 | Ru-Kat | 400 |
| 12 | 2000 | 250 | THF | 1000 | 300 | Ru-Kat | 400 |
| 13 | 2000 | 168 | THF | 1500 | 25 | Ru-Kat | 400 |
| 14 | 2000 | 168 | THF | 1500 | 50 | Ru-Kat | 400 |
| 15 | 2000 | 168 | THF | 1500 | 13 | Ru-Kat | 400 |
| 16 | 2000 | 168 | THF | 1500 | 6,4 | Ru-Kat | 400 |
| 17 | 2000 | 168 | THF | 1500 | 3) | Ru-Kat | 400 |

1) Ru-Kat.: 0,5 % Ru auf $Al_2O_3$

2) Ausbeute definiert durch Mol Produkt (= Auswaage/46) dividiert durch Mol Edukt (= Einwaage/56)

3) 24 g $(NH_4)_2CO_3$ anstatt $NH_3$

| Beisp. | Reaktionsbedingungen | | | | | Auswaage | | Ausbeute[2)] |
|---|---|---|---|---|---|---|---|---|
| | Iminierung | | Hydrierung | | | löslich | Auswaage/ | |
| | t (h) | T (°C) | t (h) | T (°C) | p (bar) | (g) | Einwaage | |
| 9 | 7 | 150 | 20 | 200 | 250 | 158 | 0,63 | 70,8 |
| 10 | 15 | 150 | 20 | 200 | 250 | 198 | 0,79 | 88,7 |
| 11 | 15 | 150 | 20 | 200 | 250 | 197 | 0,79 | 88,3 |
| 12 | 15 | 150 | 20 | 200 | 250 | 195 | 0,78 | 87,4 |
| 13 | 10 | 150 | 15 | 200 | 250 | 116 | 0,69 | 77,3 |
| 14 | 10 | 150 | 15 | 200 | 250 | 101 | 0,60 | 67,3 |
| 15 | 10 | 150 | 15 | 200 | 250 | 108 | 0,64 | 72,0 |
| 16 | 10 | 150 | 15 | 200 | 250 | 51 | 0,30 | 34,0 |
| 17 | 10 | 150 | 15 | 200 | 250 | 61 | 0,36 | 40,7 |

1) Ru-Kat.: 0,5 % Ru auf $Al_2O_3$

2) Ausbeute definiert durch Mol Produkt (= Auswaage/46) dividiert durch Mol Edukt (= Einwaage/56)

3) 24 g $(NH_4)_2CO_3$ anstatt $NH_3$

Tabelle 2b

| Stoffeigenschaften | | | | | | | | | | |
|---|---|---|---|---|---|---|---|---|---|---|
| Beispiel | Kennzahlen | | | | | | Elementaranalyse | | | |
| | OH | CO | prim. N | sek. N | tert. N | ges. N | C | H | N | O |
| | (mg KOH/g) | | (g/100 g) | | | | (g/100 g) | | | |
| 9 | 18 | 93 | 0,6 | 4,30 | 1,02 | 5,90 | n.b. | | | |
| 10 | 178 | 3 | 0,3 | 6,30 | 4,51 | 11,15 | 75,5 | 11,1 | 11,7 | 1,7 |
| 11 | 201 | <1 | 0,4 | 5,40 | 5,10 | 10,93 | 75,3 | 11,1 | 11,5 | 1,7 |
| 12 | 181 | 8 | 0,4 | 6,00 | 4,45 | 10,81 | 75,5 | 11,2 | 12,0 | 1,7 |
| 13 | 46 | 10 | 0,8 | 5,80 | 3,77 | 10,40 | n.b. | | | |
| 14 | 24 | 5 | 0,6 | 5,10 | 5,04 | 10,74 | n.b. | | | |
| 15 | 49 | 20 | 0,5 | 4,70 | 4,00 | 9,16 | n.b. | | | |
| 16 | 119 | 46 | <0,1 | 3,96 | 2,71 | 6,13 | n.b. | | | |
| 17 | 182 | 94 | <0,1 | 1,49 | 1,79 | 3,28 | n.b. | | | |
| n.b.: nicht bestimmt | | | | | | | | | | |

**Patentansprüche**

1. Polymere Polyamine erhältlich durch Umsetzung von linearen, alternierenden Kohlenmonoxid/Alk-1-en-Copolymeren mit Ammoniak oder Aminen der allgemeinen Formel (I)

$$R—NH_2 \quad (I)$$

worin R $-NH_2$, -OH, $C_1$- bis $C_{10}$-Alkyl, $C_6$- bis $C_{20}$-Aryl, $C_7$- bis $C_{20}$-Arylalkyl, $C_7$- bis $C_{20}$ Alkylaryl oder ein siliciumorganischer Rest mit 3 bis 30 Kohlenstoffatomen bedeutet

oder einem Ammoniak oder die Amine (I) freisetzenden Reagenz und anschließende Hydrierung.

2. Polymere Polyamine nach Anspruch 1, wobei das Alk-1-en Ethylen ist.

3. Polymere Polyamine nach Anspruch 1 oder 2, wobei der Gehalt an chemisch gebundenem Sauerstoff des polymeren Polyamins im Bereich von 0 bis 10 Gew.-% liegt.

4. Polymere Polyamine nach den Ansprüchen 1 bis 3, wobei deren mittleres zahlenmäßiges Molekulargewicht Mn im Bereich von 100 bis 1.000.000 liegt.

5. Verfahren zur Herstellung von polymeren Polyaminen durch Umsetzung von Kohlenmonoxid/Alk-1-en-Copolymeren mit Ammoniak oder Aminen der allgemeinen Formel (I)

$$R—NH_2 \quad (I)$$

worin R $-NH_2$, -OH, $C_1$- bis $C_{10}$-Alkyl, $C_6$- bis $C_{20}$-Aryl, $C_7$- bis $C_{20}$-Arylalkyl, $C_7$- bis $C_{20}$ Alkylaryl oder ein siliciumorganischer Rest mit 3 bis 30 Kohlenstoffatomen bedeutet

oder einem Ammoniak oder die Amine (I) freisetzenden Reagenz und anschließende Hydrierung, dadurch gekennzeichnet, daß man lineare, alternierende Kohlenmonoxid/Alk-1-en-Copolymere verwendet.

6. Verwendung der polymeren Polyamine gemäß der Ansprüche 1 bis 4 als Tenside.

7. Verwendung der polymeren Polyamine gemäß der Ansprüche 1 bis 4 als Farbübertragungsinhibitoren.

8. Verwendung der polymeren Polyamine gemäß den Ansprüchen 1 bis 4 als Dispergiermittel, Papierhilfsmittel, Schmutzlösungsmittel, Komponente in Hautcremes und Haarpflege, Vernetzer für Klebstoffe, Stabilisator für Polyoxymethylen, Korrosionsinhibitoren, Textilhilfsmittel, Hilfsmittel für Dispersionen, Klebstoffe, Schutzkolloide, Haftbeschichtung, Epoxidharzhärter in wässrigen Dispersionen, Hilfsmittel für Geschirrspülmittel, Papierhilfsmittel, Egalisiermittel für Textilien, Solubilisiator für Kosmetika, Hilfsmittel für die Metallextraktion, Komplexbildner, Kraftstoffadditiv, Schmierstoffe, Korrosionsinhibitor für wässrige Systeme, Zusatz für Leim- und Harzrohstoffe, Hilfsmittel zur Farbstoffixierung auf Textilien, Hilfsmittel in der Papierfixierung, Retentionshilfsmittel, Komplexbildner für Metall-Recycling, Stabilisatoren für Hydroxylamin.

Europäisches Patentamt

# EUROPÄISCHER RECHERCHENBERICHT

Nummer der Anmeldung
EP 97 12 2116

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
| X | US 4 322 530 A (JACHIMOWICZ) 30.März 1982<br>* Spalte 2, Zeile 65; Ansprüche *<br>--- | 1,2,5 | C08G73/02 |
| X | GB 2 136 438 A (W.R. GRACE & CO.)<br>19.September 1984<br>* Ansprüche *<br>--- | 1,5 | |
| X | US 4 028 414 A (CLOUGH) 7.Juni 1977<br>* Spalte 6, Zeile 27 - Zeile 65; Ansprüche *<br>----- | 1,3,5,6,8 | |
| | | | RECHERCHIERTE SACHGEBIETE (Int.Cl.6) |
| | | | C08G |

Der vorliegende Recherchenbericht wurde für alle Patentansprüche erstellt

| Recherchenort | Abschlußdatum der Recherche | Prüfer |
|---|---|---|
| WIEN | 19.März 1998 | WEIGERSTORFER |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)